# EUROPEAN PATENT APPLICATION

(11) **EP 4 564 363 A1**
(43) Date of publication of application: **04.06.2025**
(21) Application number: 23212565.8
(22) Date of filing: 28.11.2023
(51) Int. Cl.: G16H 30/40, G16H 40/63, G16H 50/70

(54) **METHOD, DEVICE AND SYSTEM FOR OPERATING A MEDICAL IMAGE UNIT**

(71) Applicant: Siemens Healthineers AG, 91301 Forchheim (DE)
(72) Inventor: SINAI TALAULICAR, Radhika, 560035 Bangalore (IN)
(74) Representative: Horn Kleimann Waitzhofer Schmid-Dreyer Patent- und Rechtsanwälte PartG mbB

(57) **Abstract**

The present disclosure relates to a computer-implemented method for operating a medical imaging unit configured to use a plurality of different projections, the method comprising: storing a plurality of different data sets in a database, each data set including a certain combination of limitations of a patient against the medical imaging unit and an allocated guidance information for a relative positioning between the medical imaging unit and the patient, recording a set of limitations of a certain patient against the medical imaging unit, mapping the recorded set of limitations to the database for identifying the data set in the database with the highest similarity to the recorded set of limitations, providing instructions for executing a relative positioning between the certain patient and the medical imaging unit and particularly for using a certain projection of the plurality of projections of the medical imaging unit based on the guidance information of the identified data set, and taking at least one medical image of the certain patient being positioned based on the guidance information using the medical imaging unit.

## Description

The present invention relates to a computer-implemented method, to a computer-implemented device and to a system for operating a medical image unit.

For detecting diseases of a patient, for example for detecting breast cancer, radiologists routinely perform analysis with medical imaging units to take medical images of a patient's organ, for example during mammography. Unfortunately, the taken medical images are often attributed with an inadequate quality. In consequence, the diagnosis based on the medical image may be not very accurate or not possible. Often, the process of taking the medical image has to be repeated till the desired quality and coverage of the patient's body part is achieved which is very time-consuming. Studies of the applicant have shown that the quality of the medical images is highly dependent on the relative positioning between the medical imaging unit and the patient.

When the relative positioning is not adequate, the quality of the medical images regularly suffers. Yet, to achieve a sufficient relative positioning may be challenging due to variations in the patient's body habitus, especially for unexperienced users of the medical imaging unit. When patients are suffering from psychological and/or physical limitations, like sitting in a wheelchair, the challenge of an adequate relative positioning gets further compounded. Hence, in common practice, the quality of the medical image and the required timeconsumption are heavily dependent on the experience and the skill of the user of the medical imaging unit in determining an optimum relative positioning between the medical imaging unit and the patient, based on the limitations of the patient.

Furthermore, in order to control the quality of medical images, organizations have introduced audits, wherein a user of the medical imaging unit that does not meet specific standards not just risks losing the accreditation, but also may be required to cease surgeries of the organ analyzed with the medical imaging unit. Such an organization is the MQSA (Mammography Quality Standards) division of the FDA (Food and Drug Administration) which has introduced the EQUIP auditing program (Enhancing Quality Using the Inspection Program).

Therefore, there is a need for a method and a device which enables an adequate and time-efficient usage of the medical imaging unit including the relative positioning between the medical imaging unit and the patient.

The object of the invention is therefore to provide a method, a device and a system that enables an improved operation of the medical imaging unit.

According to a first aspect, a computer-implemented method for operating a medical imaging unit configured to use a plurality of different projections is proposed. The method comprises:
storing a plurality of different data sets in a database, each data set including a certain combination of limitations of a patient against the medical imaging unit and an allocated guidance information for a relative positioning between the medical imaging unit and the patient,
recording a set of limitations of a certain patient against the medical imaging unit,
mapping the recorded set of limitations to the database for identifying the data set in the database with the highest similarity to the recorded set of limitations,
providing instructions for executing a relative positioning between the certain patient and the medical imaging unit and particularly for using a certain projection of the plurality of projections of the medical imaging unit based on the guidance information of the identified data set, and
taking at least one medical image of the certain patient being positioned based on the guidance information using the medical imaging unit.

Within the computer-implemented method, the limitations of the certain patient against the medical imaging unit are received, recorded, and mapped to the database. Hereby, the data set in the database with the highest similarity to the recorded set of limitations is identified. This identified data set may show the same limitations than the certain patient by showing a previous examination of the same patient or by showing the examination of a different patient with identical limitations. Preferably, in case no data set is found showing the same limitations than the certain patient, a data sets is identified showing similarities regarding at least such limitations, which are considered to be more determining than other limitations. Such a more determining limitation may be for example the limitation of sitting in a wheelchair. Hence, preferably, the mapping includes a limitation-specific weighting of the different limitations.

The guidance information may comprise one or more advantageous positions of the medical imaging unit, including locations and/or angular positions of a least one part of the medical imaging unit. Preferably, the guidance information may further comprise advantageous specific settings of the medical imaging unit. Further preferably, the guidance information may further comprise one or more advantageous positions, including locations and/or angular positions of the patient or at least one body part of the patient. It is further preferred that the guidance information also comprise the information which one or more projections of the medical imaging unit are optimal based on the patient's limitations. For example, in the case of a large breast size of the patient, the guidance information may be to perform an exaggerated craniocaudal lateral (XCCL) projection instead of a standard craniocaudal (CC) projection. In case the patient shows a skeletal malfunction, such as kyphosis or scoliosis, the guidance information may be to perform a reverse CC projection to achieve an adequate compression of the breast. In case the patient has a limited range of motion in the shoulder, the guidance information may be to perform a 90-degree lateral medial view and to press a film of the medical imaging unit against the sternum of the patient.

The instructions may comprise instructions of how to move and/or to position the medical imaging unit, including a translation and/or a rotation of a least one part of the medical imaging unit. Preferably, the instructions may further comprise instructions of how to adjust specific settings of the medical imaging unit. Further preferably, the instructions may further comprise instructions of how to move and/or to position the patient or at least one body part of the patient, including a translation and/or a rotation. Thus, the relative positioning between the medical imaging unit and the patient may include positioning the medical imaging unit and/or the certain patient.

With different projections is meant that medical images are taken of the same organ from varying viewing angles and/or locations and/or that medical images are taken of multiple sections of the same organ.

The database may be a computer readable database saved within the medical imaging unit, within a computer device, and/or on a network server. Hence, the database may be in contact with a plurality of the above-mentioned medical imaging units.

The medical images, for example mammography images, may be captured by and received from the medical imaging unit. The medical imaging unit may include, for example, but not limited to, magnetic resonance imaging device, computed tomography device, X-ray imaging device, ultrasound imaging device, etc. The medical images may be three-dimensional and/or related to a volume. The medical images may include one or more anatomical objects associated with the patient. The anatomical objects may be in particular one or more body parts associated with the patient that may have been imaged. The anatomical objects may include, but not be limited to, one or more imaged organs, such as the female breast. The medical images may, for example, be in the form of an array of pixels or voxels. Such arrays of pixels or voxels may be representative of intensity, absorption, or other parameter as a function of three-dimensional position, and may, for example, be obtained by suitable processing of measurement signals obtained by one or more of the above-mentioned medical imaging units. In particular, the medical image may include information associated with a region of interest for tumor detection.

By means of the present method, a fast and accurate operation of the medical imaging unit including a relative positioning between the medical imaging unit and the patient is provided. In particular, disadvantageous relative positionings and/or time-consuming trial and error procedures may be avoided by providing respective instructions. Hence, the present method may improve both the quality and the time-effort of operating the medical imaging unit, ultimately improving the derived diagnosis, and saving costs.

Preferably, the data of the certain patient including the limitations are stored in the database. This way, the database may represent an updatable knowledge repository containing positioning guidance information correlating to a variety of different combinations of limitations. The database may be updateable manually by the user of the medical imaging unit. Alternatively, or additionally, the database may be updated by a self-learning algorithm.

The database may be stored locally at the location of the medical imaging unit. Hereby, the rights to the gathered knowledge repository may be maintained more easily and safely. Alternatively, or additionally, the database may be saved on a network server, wherein the network server may be accessible or not accessible for public domain.

According to an embodiment, the guidance information and the instructions are provided within a graphical user interface.

The graphical user interface is used to enable an easy and intuitive communication between the medical imaging unit and the user of the medical imaging unit. The graphical user interface may be displayed on a screen and/or a computer monitor. It may contain data in a textual representation, images, text boxes, and/or command buttons. With the graphical user interface, the medical imaging unit may be used more time-efficiently.

According to a further embodiment, the recording of the set of limitations is performed using the graphical user interface, wherein the graphical user interface enables selecting predefined limitations and/or adding additional limitations by reading a content of a text box.

During the usage of the medical imaging unit, the medical imaging unit may receive information concerning the existence of one or more limitations of the certain patient of a list of predefined limitations. The list of predefined limitations may represent already known limitations and/or limitations which are likely to occur more often than other limitations. In addition, the medical imaging unit may receive information concerning the existence of one or more added additional limitations which are not part of the list of predefined limitations. Such added additional limitations may be received by reading the content of the text box. This way, new limitations may be received, and the graphical user interface is not limited to certain predefined limitations.

According to a further embodiment, the recording of the set of limitations is performed voice-activated using a speech recognition algorithm of a speech recognition module. This way, the time required to record the set of limitations of the certain patient may be minimized.

According to a further embodiment, the added additional limitations are added to the predefined limitations. The list of predefined limitations may be varied. For example, one or more of the added additional limitations may be added to the list of predefined limitations. Preferably, the list of predefined limitations may be structured and/or sorted. More preferably, selected ones of the list of predefined limitations may be deleted, as well. This way, the list of predefined limitations may be adjusted, for example in order to represent regionally specific and/or clientele-specific limitations and/or frequentness.

According to a further embodiment, the recorded limitations are tagged to the taken medical image and the recorded limitations are displayed together with the taken medical image, in particular using the graphical user interface.

In common practice, the limitations that led to a medical image with inadequate quality are often not documented. According to the present method, a user analyzing the taken medical image using the graphical user interface gets informed about the patient's limitations at the same time. Hereby, the user analyzing the taken medical image may be made aware of the reasons for the quality of the taken medical image and may be enabled to take a more informed decision on the image. To tag the limitations to the taken medical image may also aid during audits of organizations like the FDA by explaining the reasons that prevailed a higher quality of the medical image. In addition, the availability of both the medical image as well as the patient's limitation may enable a continuous improvement of the guidance information given by the present method.

According to a further embodiment, the relative positioning between the certain patient and the medical imaging unit is recorded using an input unit.

The input unit may comprise a video camera. The input unit may be used to record the relative positioning. The derived record may be stored along with the taken medical image within a data set of the database. This way, a continuous review of the records within the database is provided, enabling the improvement of the database and the quality of its guidance information. In consequence, the time-effort to take medical images of adequate quality may be further reduced.

According to a further embodiment, the identified data set with the highest similarity to the recorded set of limitations contains a record of the relative positioning between a patient in the data set and the medical imaging unit.

The record of the relative positioning between the patient in the data set and the medical imaging unit is saved within the database. This way, for a variety of different combinations of limitations, records showing the respective relative positioning between the patient and the medical imaging unit are available and can be found based on the selected set of limitations of the current patient.

According to a further embodiment, the instructions comprise displaying the record of the relative positioning between the patient in the data set and the medical imaging unit.

Due to the similarity of the present patient's limitations with the limitations in the identified data set, the respective record of the relative positioning between the patient in the data set and the medical imaging unit may offer a guidance for the relative positioning between the current patient and the medical imaging unit. This way, the process of the relative positioning may be further simplified and accelerated.

According to a further embodiment, providing instructions for executing the relative positioning includes providing positioning instructions in a textual representation, as an audio output, as a medical image, as a video, and/or as a step-by-step guide.

The graphical user interface may give instructions for executing the relative positioning using one or more different media formats. The graphical user interface may show the instructions as a video showing either the relative positioning of a similar patient, the same patient during a previously performed relative positioning, and/or a video showing a schematic representation of a patient. The step-by-step guide may show the relative positioning as single steps with respective videos, images, and/or text passages, wherein at a specific time, the step-by-step guide shows the next step required to perform the relative positioning. Preferably, the instructions comprise the illustration of previously taken medical images of the same patient or a different patient with similar limitations in order to illustrate the achievable quality of the medical image based on the limitations of the present patient.

According to a further embodiment, the mapping is performed in a ruled-based manner and/or a by using a trained machine learning network.

Using a ruled-based manner, the manner of how the data set with the highest similarity to the recorded set of limitations is identified may be designed purposeful. Hence, specific weightings of the different limitations may be set according to their impact on the relative positioning. Alternatively, or additionally, a machine learning network is trained such that the trained machine learning network identifies the data set in the database with the highest similarity to the recorded set of limitations. The machine learning network is a neural network, in particular a convolutional neural network (CNN), a Siamese network or a triplet network. This way, the weighing of the different limitations may be achieved automatically.

According to a further embodiment, the medical imaging unit is used to take mammography images.

Mammography is the most common medical imaging investigation used to screen for breast cancers. Hence, the quality and accuracy of the mammography image becomes quintessential in the fight against breast cancer. Yet, unlike for example in radiography, where the positioning for an examination is mostly standard irrespective of the patient's limitations, in mammography, there are multiple factors that should be considered, like the limitations of the patient against the medical imaging unit. The proposed method may improve the quality of the taken mammography images and thus may help avoiding time-consuming repetitions of taking the mammography image.

According to a further embodiment, the limitations are a combination of at least one of the following: size of breasts, patient with limited range of motion in the shoulder, patient on a wheelchair, patient with paralysis, patient with large abdomen, fragile and/or unstable patient, patient with low balance, patient sensitive to compression, presence of medical devices, patient having undergone a recent lumpectomy, mastectomy, or other surgery, patient with skeletal malformations such as kyphosis, scoliosis, lordosis.

The set of limitations may be different for different modalities, like for taking X-Ray or CT images.

According to a second aspect, a computer-implemented device for operating a medical imaging unit is provided. The computer-implemented device comprises:
one or more processing units,
a network interface which is configured to receive one or more medical images captured by a medical imaging unit, and
a memory coupled to the one or more processing units,
the memory comprising a module configured to perform the method steps according to the first aspect or of any embodiment of the first aspect.

The respective unit, e.g., the processing unit or the network interface, may be implemented in hardware and/or in software. If said unit is implemented in hardware, it may be embodied as a device, e.g., as a computer or as a processor or as a part of a system, e.g., a computer system. If said unit is implemented in software, it may be embodied as a computer program product, as a function, as a routine, as a program code or as an executable object. The computer-implemented device may be referred to as position guidance module.

The embodiments and features according to the first aspect are also embodiments of the second aspect.

According to a third aspect, a system for operating a medical imaging unit is provided. The system comprising:
one or more computer-implemented devices,
the medical imaging unit coupled to the one or more computer-implemented devices,
the one or more computer-implemented devices comprising instructions, which when executed causes the one or more computer-implemented devices to perform the method steps according to the first aspect or of any embodiment of the first aspect.

The embodiments and features according to the first aspect are also embodiments of the third aspect.

According to a fourth aspect, a computer program product is provided. The computer program product comprising machine readable instructions, that when executed by one or more processing units, cause the one or more processing units to perform the method steps according to the first aspect or of any embodiment of the first aspect.

The embodiments and features according to the first aspect are also embodiments of the fourth aspect.

A computer program product, such as a computer program means, may be embodied as a memory card, USB stick, CD-ROM, DVD or as a file which may be downloaded from a server in a network. For example, such a file may be provided by transferring the file comprising the computer program product from a wireless communication network.

According to a fifth aspect, a computer readable medium on which program code sections of a computer program are saved is provided. The program code sections being loadable into and/or executable in a system to make the system execute the method steps according to the first aspect or of any embodiment of the first aspect when the program code sections are executed in the system.

The embodiments and features according to the first aspect are also embodiments of the fifth aspect.

The realization by a computer program product and/or a computer-readable medium has the advantage that already existing management systems can be easily adopted by software updates in order to work as proposed by the invention.

Further possible implementations or alternative solutions of the invention also encompass combinations - that are not explicitly mentioned herein - of features described above or below with regard to the embodiments. The person skilled in the art may also add individual or isolated aspects and features to the most basic form of the invention.

Further embodiments, features, and advantages of the present invention will become apparent from the subsequent description and dependent claims, taken in conjunction with the accompanying drawings, in which:
- FIG. 1: illustrates a block diagram of a system for operating a medical imaging unit,
- FIG. 2: illustrates a block diagram of a computer-implemented device for operating the medical imaging unit,
- FIG. 3: illustrates a flowchart of a method for operating the medical imaging unit,
- FIG. 4: shows a schematic illustration of a first output of a graphical user interface for recording a set of limitations of a certain patient,
- FIG. 5: shows a schematic illustration of a second output of the graphical user interface providing guidance information and instructions to a user of the medical imaging unit,
- FIG. 6: shows a schematic illustration of a third output of the graphical user interface providing a display of the medical image of the certain patient together with the patient's limitations.

Hereinafter, embodiments for carrying out the present invention are described in detail. The various embodiments are described with reference to the drawings, wherein like reference numerals are used to refer to like elements throughout. In the following description, for purpose of explanation, numerous specific details are set forth in order to provide a thorough understanding of one or more embodiments. It may be evident that such embodiments may be practiced without these specific details.

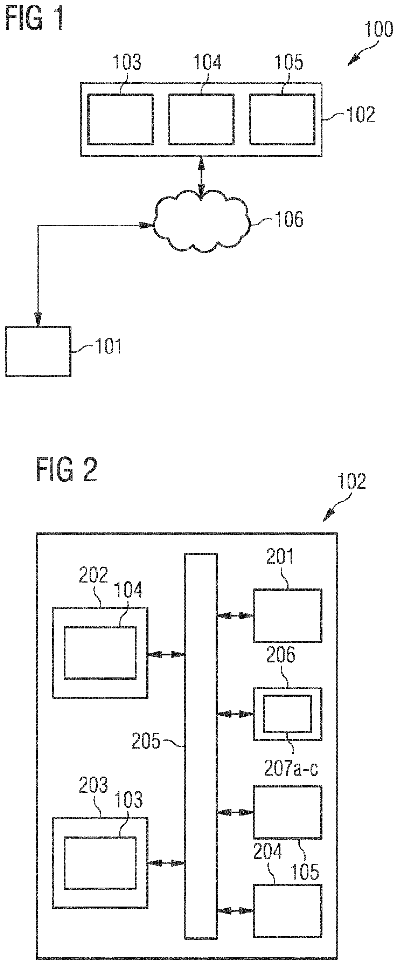

FIG. 1 provides an illustration of a block diagram of a system 100 comprising a medical imaging unit 101. The medical imaging unit 101 is capable of acquiring a plurality of medical images. The medical imaging unit 101 may be, for example, a scanner unit such as a magnetic resonance imaging unit, a computed tomography imaging unit, an X-ray fluoroscopy imaging unit, an ultrasound imaging unit, etc. In the illustrated embodiment, the medical imaging unit 101 is a device capable of taking mammography images.

In addition, the system 100 comprises a computer-implemented device 102. The computer-implemented device 102 may be referred to as positioning guidance module. The computer-implemented device 102 includes a database 103 that comprises a plurality of data sets, wherein each data set comprises a medical image, a combination of at least one limitation of a patient against the medical imaging unit, and respective guidance information regarding a relative positioning between the patient and the medical imaging unit. In an embodiment, the database 103 may comprise medical images captured by a MR scanner, by a CT scanner, by an X-ray scanner and/or by an ultrasonic scanner. In the shown embodiment, the database 103 comprises mammography images. The computer-implemented device 102 includes a computer program product 104 comprising machine readable instructions that when executed cause the recording of a set of limitations of a certain patient and the providing of guidance information with respect to the limitations of the certain patient. Additionally, the computer-implemented device 102 includes a network interface 105 for enabling a communication with the medical imaging unit 101 via a network 106.

The network may be, for example, a local area network (LAN), a wide area network (WAN), or a WiFi, etc. In one embodiment, the computer-implemented device 102 is deployed in a cloud computing environment. As used herein, "cloud computing environment" refers to a processing environment comprising configurable computing physical and logical resources, for example, networks, servers, storage, applications, services, etc., and data distributed over the network 106, for example, the internet. The cloud computing environment provides on-demand network access to a shared pool of the configurable computing physical and logical resources.

The computer-implemented device 102 is used by users, for example, medical personnel such as a radiologist, physician, etc. In an embodiment, the computer-implemented device 102 may be used by the user to receive medical images associated with the patient from the database 103.

FIG. 2 is a block diagram of the computer-implemented device 102. The computer-implemented device 102 comprises a processing unit 201, a memory 202, a storage unit 203, an input unit 204, a bus 205, an output unit 206, and the network interface 105.

The processing unit 201, as used herein, means any type of computational circuit, such as, but not limited to, a microprocessor, microcontroller, complex instruction set computing microprocessor, reduced instruction set computing microprocessor, very long instruction word microprocessor, explicitly parallel instruction computing microprocessor, graphics processor, digital signal processor, or any other type of processing circuit. The processing unit 201 may also include embedded controllers, such as generic or programmable logic devices or arrays, application specific integrated circuits, single-chip computers, and the like.

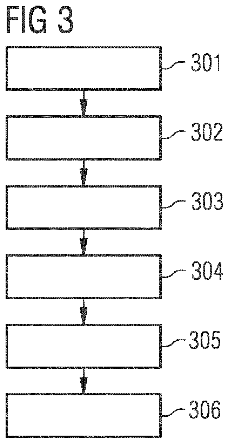

The memory 202 may be volatile memory and non-volatile memory. The memory 202 may be coupled for communication with said processing unit 201. The processing unit 201 may execute instructions and/or code stored in the memory 202. A variety of computer-readable storage media may be stored in and accessed from said memory 202. The memory 202 may include any suitable elements for storing data and machine-readable instructions, such as read only memory, random access memory, erasable programmable read only memory, electrically erasable programmable read only memory, a hard drive, a removable media drive for handling compact disks, digital video disks, diskettes, magnetic tape cartridges, memory cards, and the like. In the present embodiment, the memory 202 comprises the computer program product 104 stored in the form of machine-readable instructions on any of said above-mentioned storage media and may be in communication to and executed by the processing unit 201. When executed by the processing unit 201, the computer program product 104 causes the processing unit 201 to record the set of limitations of the certain patient, to map the limitations to a data set of the database 103 showing the highest similarity with the limitations of the certain patient, to provide guidance information to the user, and to take a medical image using the medical imaging unit 101. Method steps executed by the processing unit 201 to achieve the abovementioned functionality are elaborated upon in detail in FIG. 3.

The storage unit 203 may be a non-transitory storage medium which stores the database 103. The input unit 204 may include input means such as keypad, touch-sensitive display, and/or camera. With the input unit 204, a record of the relative positioning of the certain patient may be captured. With the output unit 206, data may be shown to the user, possibly using a TV screen, a computer monitor, and/or other displays. The bus 205 acts as interconnect between the processor 201, the memory 202, the storage unit 203, the input unit 204, the output unit 206 and the network interface 105.

Those of ordinary skilled in the art will appreciate that said hardware depicted in FIG. 1 and/or 2 may vary for particular implementations. For example, other peripheral devices such as an optical disk drive and the like, Local Area Network (LAN)/ Wide Area Network (WAN)/ Wireless (e.g., Wi-Fi) adapter, graphics adapter, disk controller, input/output (I/O) adapter also may be used in addition or in place of the hardware depicted. Said depicted example is provided for the purpose of explanation only and is not meant to imply architectural limitations with respect to the present disclosure.

The computer-implemented device 102 may comprise an operating system employing graphical user interfaces 207a - c. The graphical user interfaces 207a - c are displayed by the output unit 206. Said operating system permits multiple display windows to be presented in the graphical user interfaces 207a - c simultaneously with each display window providing an interface to a different application or to a different instance of the same application. A cursor in said graphical user interface may be manipulated by a user through a pointing device. The position of the cursor may be changed and/or an event such as clicking a mouse button, generated to actuate a desired response.

One of various commercial operating systems, such as a version of Microsoft Windows^{™}, a product of Microsoft Corporation located in Redmond, Washington may be employed if suitably modified. Said operating system is modified or created in accordance with the present disclosure as described. Disclosed embodiments provide systems and methods for processing medical images.

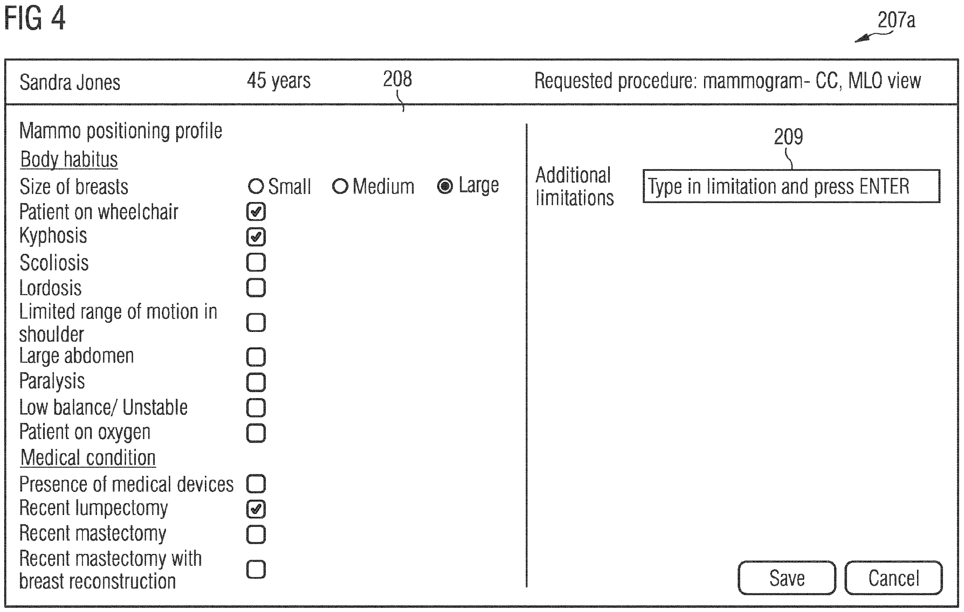

FIG. 3 illustrates a flowchart of a method for operating the medical imaging unit. In particular, the method of FIG. 3 may be executed by the above-discussed processing unit 201. The embodiment of FIG. 3 includes the method steps 301 - 306:
In step 301, a plurality of different data sets is stored in the database 103. Each data set includes a combination of at least one limitation of a patient against the medical imaging unit 101 and an allocated guidance information for a relative positioning between the medical imaging unit 101 and the patient. Each data set includes a record of the relative positioning between the certain patient and the taken medical image, as well.
In step 302, a set of limitations of the certain patient against the medical imaging unit 101 is recorded using a first output of the graphical interface 207a. The limitations may be limitations of the certain patient against mammography. The first output of the graphical user interface 207a is elaborated upon in detail in FIG. 4.
In step 303, the recorded set of limitations is mapped to the database 103 for identifying the data set in the database 103 with the highest similarity to the recorded set of limitations. The mapping may be performed either in a ruled-based manner and/or by using a trained machine learning network. The mapping may include identifying the data set in the database 103 showing the highest number of similar limitations than the certain patient. Hereby, the mapping may show respect to a weighting of the limitations to render certain limitations more important to map with the limitations of the certain patient than other limitations. An example for a limitation with a higher weighting may be sitting in a wheelchair. The machine learning network may be a neural network, in particular a convolutional neural network (CNN), a Siamese network or a triplet network.

In the training phase, the machine learning network is trained by entering information about certain patients with specific combinations of limitations together with corresponding guidance information to perform the relative positioning. Preferably, the machine learning network is trained during the usage of the medical imaging unit 101, possibly while using the ruled-based manner.

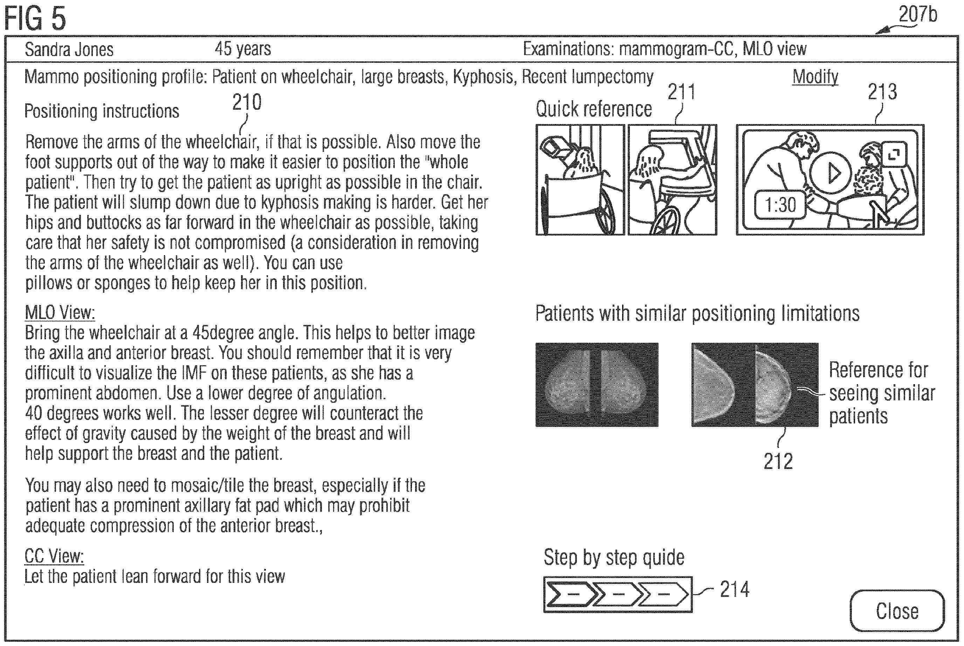

In step 304, instructions are provided for executing a relative positioning between the certain patient and the medical imaging unit 101 and for using a certain projection of the plurality of projections of the medical imaging unit 101 based on the guidance information of the identified data set. The instructions may include instructions to position and/or to rotate at least one part of the medical imaging unit 101 and/or to adjust specific settings of the medical imaging unit 101. The instructions may further include instructions to position and/or to rotate at least one body part of the patient. The instructions may further include a medical image taken during a previous usage of the medical imaging unit 101. The instructions are provided using a second output of the graphical user interface 207b. The second output of the graphical user interface 207b is elaborated upon in detail in FIG. 5.

In step 305, at least one medical image of the certain patient or of at least one body part of the certain patient being positioned based on the guidance information is taken using the medical imaging unit 101. In particular, the at least one medical image may be acquired by an MR scanner, a CT scanner, an X-ray scanner, or an ultrasonic scanner. In the shown embodiment, the medical image is a mammography image. In case additional limitations are noticed during the procedure of taking the medical image, the recorded set of limitations is updated respectfully. After taking the medical image, the recorded set of limitations of the certain patient is tagged to the taken medical image. Both the medical image as well as the recorded set of limitations may be stored as one file and/or within the same data set in the database 103.

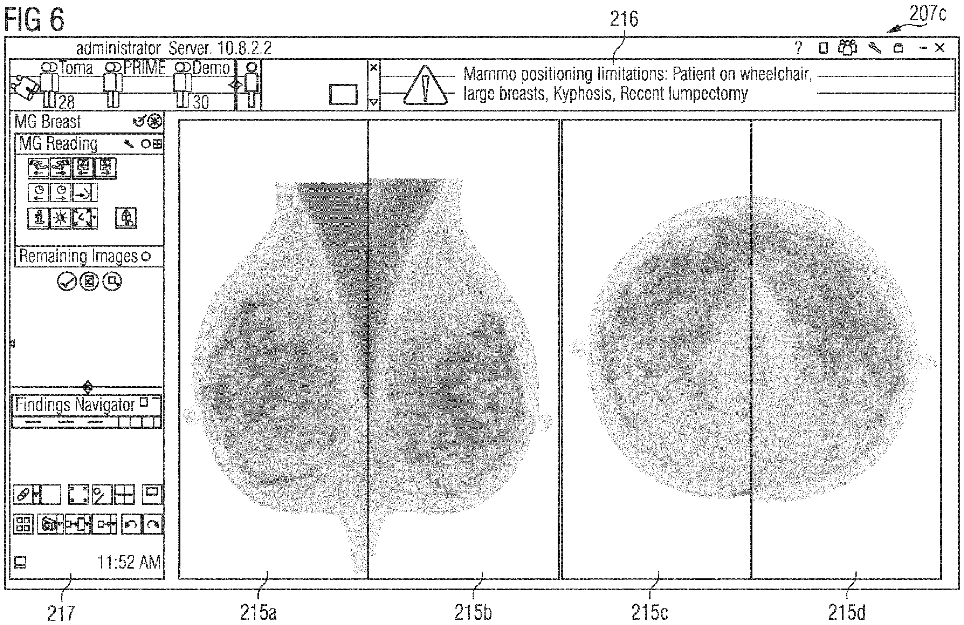

In step 306, the taken medical image is displayed to a user together with the recorded set of limitations of the certain patient using a third output of the graphical user interface 207c. A user of the medical imaging unit, possibly a radiologist and/or physician, may review both the medical image and the limitations at the same time. The third output of the graphical user interface 207c is elaborated upon in detail in FIG. 6. It is emphasized that the step 306 is optional and not required for the present method.

FIG. 4 shows a schematic illustration of the first output of the graphical user interface 207a. It enables the recording of a set of limitations of the certain patient. The first output of the graphical user interface 207a offers the user to choose limitations from a list 208 with a number of predefined limitations which are likely to occur. In addition, the first output of the graphical user interface 207a provides a text box 209 for entering additional limitations which are not part of the list 208 of predefined limitations.

FIG. 5 shows a schematic illustration of the second output of the graphical user interface 207b. It provides guidance information and instructions 210 in textual representation to the user to perform the relative positioning between the medical imaging unit 101 and the certain patient with respect to the limitations of the certain patient. The guidance information and instructions may also include an image 211. The second output of the graphical user interface 207b may further show a medical image 212 and/or a record 213 in the form of a video of the relative positioning between the certain patient and/or a similar patient and the medical imaging unit 101 from previous usages of the medical imaging unit 101. Furthermore, the second output of the graphical user interface 207b may show a step-by-step guide 214 illustrating the respective next step in order to perform the relative positioning.

FIG. 6 shows a schematic illustration of the third output of the graphical user interface 207c. The third output of the graphical user interface 207c provides an illustration of at least one taken medical image 215a - d of the certain patient, possibly showing mammography images being taken using two different projections. Furthermore, third output of the graphical user interface 207c provides a list 216 of the recorded patient's limitations. This enables the user to mention the limitations in a report including the derived diagnosis. A tool box 217 provides a number of controls to navigate through the medical images 215a - d, to analyze them and/or to edit them. Furthermore, the tool box 217 enables to update the recorded list of limitations.

The foregoing examples have been provided merely for the purpose of explanation and are in no way to be construed as limiting of the present invention disclosed herein. While the invention has been described with reference to various embodiments, it is understood that the words, which have been used herein, are words of description and illustration, rather than words of limitation. Further, although the invention has been described herein with reference to particular means, materials, and embodiments, the invention is not intended to be limited to the particulars disclosed herein, rather, the invention extends to all functionally equivalent structures, methods and uses, such as are within the scope of the appended claims. Those skilled in the art, having the benefit of the teachings of this specification, may effect numerous modifications thereto and changes may be made without departing from the scope and spirit of the invention in its aspects.

### REFRENCE SIGNS

- 100: system
- 101: medical imaging unit
- 102: computer-implemented device
- 103: database
- 104: computer program product
- 105: network interface
- 106: network
- 201: processing unit
- 202: memory
- 203: storage unit
- 204: input unit
- 205: bus
- 206: output unit
- 207a - c: graphical user interface
- 208: list of predefined limitations
- 209: text box
- 210: instructions in textual representation
- 211: instructions as image
- 212: medical image
- 213: record
- 214: step-by-step guide
- 215a - d: medical image
- 216: list of limitations
- 217: tool box
- 301 - 306: method steps

## Claims

1. A computer-implemented method for operating a medical imaging unit (101) configured to use a plurality of different projections, the method comprising:
storing (301) a plurality of different data sets in a database (103), each data set including a certain combination of limitations of a patient against the medical imaging unit (101) and an allocated guidance information for a relative positioning between the medical imaging unit (101) and the patient,
recording (302) a set of limitations (216) of a certain patient against the medical imaging unit (101),
mapping (303) the recorded set of limitations (216) to the database (103) for identifying the data set in the database (103) with the highest similarity to the recorded set of limitations (216),
providing (304) instructions (210 - 214) for executing a relative positioning between the certain patient and the medical imaging unit (101) and particularly for using a certain projection of the plurality of projections of the medical imaging unit (101) based on the guidance information of the identified data set, and
taking (305) at least one medical image (215a - d) of the certain patient being positioned based on the guidance information using the medical imaging unit (101).

2. The method according to claim 1, wherein the guidance information and the instructions (210 - 214) are provided within a graphical user interface (207b).

3. The method according to claim 2, wherein the recording (302) of the set of limitations (216) is performed using the graphical user interface (207a), wherein the graphical user interface (207a) enables selecting predefined limitations (208) and/or adding additional limitations by reading a content of a text box (209).

4. The method according to claim 3, wherein the added additional limitations are added to the predefined limitations (208).

5. The method according to one of claims 1 to 4, wherein the recorded limitations (216) are tagged to the taken medical image (215a - d) and the recorded limitations (216) are displayed together with the taken medical image (215a - d), in particular using the graphical user interface (207c).

6. The method according to one of claims 1 to 5, wherein the relative positioning between the certain patient and the medical imaging unit (101) is recorded using an input unit (204).

7. The method according to one of claims 1 to 6, wherein the identified data set with the highest similarity to the recorded set of limitations (216) contains a record (213) of the relative positioning between a patient in the data set and the medical imaging unit (101).

8. The method according to claim 7, wherein the instructions (210 - 214) comprise displaying the record (213) of the relative positioning between the patient in the data set and the medical imaging unit (101).

9. The method according to one of claims 1 to 8, wherein providing (304) instructions for executing the relative positioning includes providing positioning instructions in a textual representation (210), as an audio output, as a medical image (212), as a video (213), and/or as a step-by-step guide (214).

10. The method according to one of claims 1 to 9, wherein the mapping (303) is performed in a ruled-based manner and/or a by using a trained machine learning network.

11. The method according to one of claims 1 to 10, wherein the medical imaging unit (101) is used to take mammography images.

12. The method according to one of claims 1 to 11, wherein the limitations are a combination of at least one of the following: size of breasts, patient with limited range of motion in the shoulder, patient on a wheelchair, patient with paralysis, patient with large abdomen, fragile and/or unstable patient, patient with low balance, patient sensitive to compression, presence of medical devices, patient having undergone a recent lumpectomy, mastectomy, or other surgery, patient with skeletal malformations such as kyphosis, scoliosis, lordosis.

13. A computer-implemented device (102) for operating a medical imaging unit (101), the computer-implemented device (102) comprising:
one or more processing units (201),
a network interface (105) which is configured to receive one or more medical images (215a - d) captured by a medical imaging unit (101), and
a memory (202) coupled to the one or more processing units (201), the memory (202) comprising a module configured to perform the method steps (301 - 305) as claimed in any one of claims 1 to 12.

14. A system (100) for operating a medical imaging unit (101), the system (100) comprising:
one or more computer-implemented devices (102),
the medical imaging unit (101) coupled to the one or more computer-implemented devices (102),
the one or more computer-implemented devices (102) comprising computer program product (104), which when executed causes the one or more computer-implemented devices to perform the method steps (301 - 305) as claimed in any one of claims 1 to 12.

15. A computer program product (104) comprising machine readable instructions, that when executed by one or more processing units (201), cause the one or more processing units (201) to perform the method steps (301 - 305) according to claims 1 to 12.
